# EUROPEAN PATENT SPECIFICATION

(11) **EP 1 604 640 B1**
(45) Date of publication and mention of the grant of the patent: **07.03.2012**
(21) Application number: 04010246.9
(22) Date of filing: 30.04.2004
(51) Int. Cl.: A61Q 5/00, A61K 8/92, A61K 8/81

(54) **Treatment composition for hair and scalp**
Mittel zur Behandlung der Haare und der Kopfhaut
Composition de traitement des cheveux et du cuir chevelu

(43) Date of publication of application: 14.12.2005
(73) Proprietor: KPSS-Kao Professional Salon Services GmbH, 64297 Darmstadt (DE)
(72) Inventor: Hoffmann, Martin, 64673 Zwingenberg (DE); Schupp, Bettina, 64404 Bickenbach (DE)

(56) References cited:
- DE-A- 10 150 729
- US-A1- 2003 129 268

## Description

This invention relates to a hair and scalp treatment composition comprising butyl esters of fatty acids of avocado oil, which is know as 5-α-avocuta. The compositions show primarily excellent anti-fat effect owing to their 5-α-avocuta content and in combination with additional active ingredients as well excellently suitable for use in curing other scalp related cosmetic problems. Beneficial effects of the composition of the present invention for hair are excellent caring, improvement in combing, shine and volume.

Antidandruff hair and scalp compositions have gained much attention on the market. Many of these products come in the form of shampoos or hair tonics which contain anti-dandruff ingredients such as zinc pyrithione, triclosan, octopirox, salicylic acid, climbazol and similar other materials.

Additionally scalp tonics are available on the market as well with moisturizing effect. They comprise in addition to the common cosmetic ingredients moisturizing agents, which prevents drying out of hair and scalp.

Greasy hair is a well-known problem due to the high sebum activity on the scalp found out in consumer surveys. There are compositions available on the consumer market claiming solving the problem. Zinc salts such as zinc salt of pyrrolidoncarboxylic acid (PCA) has been used for its anti-fat effect in hair and scalp cleansing and/or scalp care products. The effect obtained with this ingredient has found to be limited, so that new developments with improved performance are therefore desirable.

Thus, the objective of the present invention is finding out composition suitable for using as hair and scalp treatment with excellent anti-fat effect. It has surprisingly been found out that incorporation of butyl esters of fatty acids of avocado oil, which is known as 5-α-avocuta, into hair and scalp treatment compositions excellent anti-fat effect is observed. The anti fat effect of the compositions is determined by a use test with end users, admitting having greasy hair and scalp and using normally anti-fat hair and scalp treatment products claiming the anti-fat effect.

Butyl esters of fatty acids of avocado oil is prepared from natural avocado oil by first obtaining fatty acids and subsequent esterifying. Main fatty acid component in 5-α-Avocuta is oleic acid. It comprises furthermore palmitic, palmitoleic and linoleic acids. It comprises mainly unsaturated fatty acids.

According to the invention, hair and scalp treatment composition characterized in that it comprises butyl esters of fatty acids of avocado oil one or more thickening polymer, and one or more moisturising agent selected from polyols.

Hair and scalp treatment composition of the present invention comprises butyl esters of fatty acids of avocado oil (5-α-Avocuta) at a concentration of 0.01 to 5%, preferably 0.01 to 2%, more preferably 0.01 to 1% by weight calculated to total composition.

Hair and scalp treatment compositions of the present invention comprise one or more thickener(s). Suitable thickeners are selected from natural and synthetic polymers or their mixtures. Natural polymers are typically cellulose and its derivatives such as hydroxyethylcellulose, hydroxypropyl cellulose, guar gum, chitosan and xanthan gum.

Synthetic polymers are suitably selected from non-ionic, anionic, cationic and amphoteric ones and their combinations. The most preferred ones among them are anionic and the cationic ones because of their thickening performance. In principal any anionic polymer is suitable for use in the compositions of the present invention. In practice most preferred ones are acrylate type of anionic polymers and their derivatives. Those are for example known with the trade name Carbopol from Goodrich. For the best thickening performance, those polymers should be neutralised using an alkaline solution after dispersing in aqueous medium. In the case that composition is thickened with an acrylate polymer, attention should be paid to their compatibility with cationic ingredients and electrolytes. Preferably, acrylate type of anionic polymers should not be present in the same composition with cationic compounds and electrolyte. Minor electrolyte (salt) quantities for example for adjusting pH is possible.

Cationic polymers are of greater variability in their structure found to be suitable for compositions of the present invention. It has been found out that especially those of cationic cellulose derivatives known as Polymer JR type from Amerchol such as Polyquaternium 10 or cationic guar gum known with trade name Jaguar from Rhône-Poulenc and chemically for example Guar hydroxypropyl trimonium chloride, are preferred ones.

Furthermore, it has especially been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Amphoteric or zwitterionic polymers, preferably be used in mixture with at least one non-ionic and/or cationic polymers, are also found to be suitable for compositions of the present invention. Examples to the preferred ones are copolimerisate of n-octylacrylamide, acrylic or metahcrylic acid and tert.-butylaminoethylmethacrylate known with its trade name Amphomer, copolymer of methacryloylethylbetaine and alkyl methacrylate known as Yukaformer, terpolymer of metahcrylic or acrylic acid and itaconoic acid and a basic monomer of mono or dialkylaminoalkyl acrylate or methacrylate or acrylate of methacrylamide known with the trade name Aquaflex SF 40.

As amphoteric polymers which can be used alone or in mixture with at least one additional cationic and/or nonionic polymer, special reference is here made in particular to copolymers of N-octyl acrylamide, (meth)acrylic acid and tert.-butyl-aminoethyl methacrylate of the type "Amphomer®"; copolymers from methacryloyl ethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl aminoalkyl (meth)acrylates or mono- or dialkyl aminoalkyl (meth)acrylamides containing basic groups, in particular amino groups; copolymers from N-octyl acrylamide, methyl methacrylate, hydroxypropyl-methacrylate, N-tert.-butyl aminoethyl methacrylate and acrylic acid, as well as the copolymers known from US-A 3,927,199.

Further anionic polymers are suitable for compositions of the present invention are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g., "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®".

Concentration of those polymers of anionic, cationic, non-ionic and/or amphoteric or zwitterionic character is generally in the range of 0.01 - 5%, preferably 0.05 - 3% and most preferably 0.05 - 2% by weight, calculated to the total composition.

It should as well be noted here that the polymers primarily used for thickening compositions of the present invention may have certain level of styling, setting, effect.

This is found to be especially beneficial because of preferably leave in application of the compositions of the present invention, and therefore, allowing hair styling without use of additional products hereto.

Hair and scalp treatment composition of the present invention can be in any form of application, e.g. as solutions, emulsions, dispersions, gels or aerosol foams , preferably as hair and scalp tonics or lotions. In the case that aerosol type of application is preferred, compositions comprise then additionally cosmetically acceptable pressurized gas such as dimethylether, short chain alkanes, isoalkanes or halo alkanes. The hair and scalp treatment compositions of the present invention are directly applied to the scalp and preferably not rinsed off. In certain case it may be necessary to use so-called intensive or concentrated (high active ingredient comprising compositions) composition on hair and scalp and after certain processing time, the hair and scalp can be clarified with water. This form of application can be preferred when the problem is aggravated.

Compositions of the present invention may comprise components improving scalp feeling in addition to those moisturizing agents. Those are the cooling agents selected from the well known ones menthol or mentyl lactate or their mixtures. Certainly, the used solvents such as ethanol have also been proven to show cooling effect on skin. According to the present invention it has been found out that in the presence of one or more above-mentioned cooling agents, perceivable effect is understood to be much higher. The total concentration of the cooling agents, menthol or menthyl lactate or their mixtures, is in the range of 0.05 to 1%, preferably 0.05 to 0.75% by weight calculated to the total weight of the composition.

Hair and scalp treatment compositions of the present invention comprises moisturizing ingredients selected from polyols such as propanediols, glycerol, polyethylene glycols, propyleneglycol, 1,3-propyleneglycol or butyleneglycol. The concentration of those can be in the range of 0.05 - 5%, preferably 0.05 - 3% and most preferably 0.05-2% by weight calculated total composition.

Scalp treatment composition of the present invention can comprise fragrance components. The fragrance component is not obligatory, and especially in the case of hypoallergenic compositions it should be excluded. This means, according to the present invention the compositions are both with and without fragrance. If the fragrance is present the concentration should be below 0.5% by weight calculated total composition.

Preservatives or antimicrobial agents can as well be part of the compositions. Here again in the case of hypoallergenic (allergen level is reduced to practically zero, or no allergen is added) compositions preservatives should be excluded. Especially for compositions packed in the form of an aerosol, preservative is not needed.
Depending on the preservative, the concentration should be adjusted for the optimum effect.

Needs of hair and scalp can certainly not be limited to a single problem. Therefore, it is often needed multipurpose products such as and anti fat hair and scalp cleansing composition with UV filter for protection hair and scalp from damaging effects of UV rays as well with an antidandruff ingredient for solving problems of greasy hair and scalp and with dandruff.

Antidandruff agents such as piroctone olamine (Octopirox), zinc pyrrithion, climbazol can as well be used when an antidandruff effects is targeted. Typical useful concentration range for antidandruff agents is between 0.1 - 2% by weight calculated to the total composition.

Compositions of the present invention can comprise UV filters either for stabilization of the product colour or for protection of hair from environmental influences such as loss of elasticity, loss of hair colour (bleaching effect of sun light). The UV-absorbing substance is preferably selected from the following compounds:
4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy-benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher. The amount of the UV-absorber ranges from 0.01 to 10% by weight, preferably 0.01 to 5%, more preferably from 0.05 % to 1 % by weight, calculated to the total composition.

Organic solvents can be incorporated into the compositions of the present invention. Proffered are, without bringing any limitation, ethanol, propanol, isopropanol. Concentration of organic solvent should not exceed 40%, preferably 30% and more preferably 25% by weight calculated to total composition.

Solubilizers may be added to the compositions especially when oily substances are chosen as conditioning agents and fragrance oils with highly lipophilic properties. In principal any surfactant is suitable as solubilizer. In practice the most preferred ones are typical hydrogenated castor oil known with the trade mark Cremophor RH series from BASF. Typical concentration of the solubilizers can be in the range of 0.01 - 2% by weight, preferably 0.1 - 1 % by weight, calculated to the total composition.

The pH of the hair and scalp treatment compositions according to the present invention is in the range of 3 to 8, preferably 3 to 7, more preferably 4 to 7. For adjusting the pH of the said compositions, following ingredients can be used: Organic acids such as citric acid, lactic acid, tartaric acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. The pH of the composition can also be adjusted to the required pH by using alkaline solution such as sodium hydroxide, potassium hydroxide or their salts with those acids mentioned above in the case that at the selected acid concentration pH of the composition is lower than that of the aimed value.

Additionally natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves and white hawthorn.

Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4^{th} Ed..

Among the natural ingredients in the form of an extract, preferred component of the composition according to the invention is green tea extract. This tea extract is obtained from the leaves, leaf buds and tender stems of the tea shrub, Camellia sinensis or Camellia oleifera, by aqueous or hydro-alcoholic extraction and subsequent spray-drying. In difference to black tea, green tea is a non-fermented product obtained from the Thea sinensis or Thea assamica species. An overview of the biological and pharmacological effects of green tea and the ingredients thereof can be found, e.g., in an article by A. Pistorius, "Seifen-Öle-Fette-Wachse-Journal", Volume 122., No. 7/1996, pages 468 to 471, to which reference is made. The content of green tea extract is variable in the compositions according to the invention. It preferably ranges from 0.01 % to 10 %, preferably 0.05 % to 5% by weight, calculated to the total composition and the pulverulent extract.

Another preferred natural ingredient in the form of an extract is oatmeal extract because of its soothing and anti-itchy effects. It is especially preferred as by frequent wash of greasy hair and scalp with cleansing preparation lead often drying out effect, which then causes itchiness. In order to reduce the unpleasant dry feeling and therefore itchiness, use of oatmeal extract is preferred. The use of oatmeal and its extracts in cosmetic compositions is principally already known from European Patent Applications EP 619 950, EP 620 979 and EP 739 621. The oatmeal extract used according to the invention at a concentration of 0.01 to 2% by weight calculated to total composition and dry residue of the extract.

Natural oils are as well suitable for their conditioning effects on hair and scalp. Suitable ones without limitation are such as olive oil, almond oil, avocado oil, weizenkeim oil, ricinus oil, coconut oil, palm oil, sesame oil, peanut oil, whale oil, sunflower oil, peach kernel oil, wheat germ oil, macadamia nut oil, night primrose oil, jojoba oil, castor oil and soya oil.

The viscosity of the compositions is usually below 2500 mPa.s measured at at 20°C, with a Brookfield or Höppler at a shear rate of 10 sec⁻¹. Viscosity of the composition should be chosen in accordance with the packaging to be used.

Furthermore, hair and scalp treatment compositions of the present invention can comprise all substances customarily found in such preparations. Examples of such substances are complexing agents and dyestuffs.

In practice, the compositions of the present invention can as well be used together with a cleansing composition designed preferably for the same purpose. For this purpose, compositions of the present invention can appropriately be offered in a kit. Then, hair and scalp treatment kit comprises 2 components component A and component B and component A is being the cleansing composition comprising at least one surfactant selected form anionic, nonionic and amphoteric ones and at least one hair conditioning ingredient and optionally comprises 5-a-avocuta and component B is being a hair and scalp treatment composition according to present invention disclosed above.

Accordingly, kit is applied in a process that first component A being a cleansing composition is applied on dry or wet hair and massaged thoroughly and left on hair and scalp for a period of 1 to 20 min, and rinsed off with water and subsequently component B being a hair and scalp treatment composition according to the present invention is applied onto hair and scalp and not rinsed off.

As component A in principal any cleansing composition designed for hair and scalp wash can be used. Suitable, cleansing compositions comprise one or more cleansing surfactant selected from anionic, nonionic and amphoteric ones and at least one hair conditioning agent. Some of those surfactant without limiting the choices alkyl polyglucosides, betaine type of surfactants and ethoxylated fatty alcohol sulfates. As conditioning agents again without limitation, suitable are any cationic polymer mentioned above, cationic surfactants with one or more alkyl chains with or without ester and amide bonds in the chains, oily substances being natural and/or synthetic ones.

The following examples illustrate the invention.

### Example 1

| **Soothing hair and scalp lotion** | |
|---|---|
| Polyquaternium - 37 | 0.3% by weight |
| Ethanol | 20 |
| 5-α-avocuta | 0.3 |
| Cremophor RH 40 | 0.3 |
| Glycerin | 0.5 |
| Water | q.s. to 100 |

Polyquaternium-37 is dissolved in sufficient amount of water and combined with 5-α-avocuta and Cremophor RH 40 (solubilizer) mixture and finally ethanol, glycerin is added and is made up to 100% with water.

The composition is tested for demonstrating its anti fat effect in a use test with 10 end users admitting having greasy hair and using normally anti-fat hair and scalp treatment products claiming the anti-fat effect. In a control group quantitatively the same composition as above but not containing 5-α-Avocuta is tested in the same way. All participants, male, aging 20 to 45, 10 in each group, are asked to use the hair and scalp treatment composition for the period of 3 weeks. Prior to the test period their washing habits are evaluated. Average hair and scalp washing frequency is 6 in a week. The test persons are asked to continue using their own shampoo (hair cleansing products).

After 3 week of usage, average wash frequency in the group using the composition according to the example 1 was dropped to 3 in a week on average (most of the test persons answered the question as every other day) whereas in the group using the composition not containing 5-α-Avocuta (non-inventive composition) did not change at all, the average number was 6 (most of the test persons answered the question as every day). In the group using the inventive composition, preference was approximately 90% towards the inventive composition when compared to hair and scalp treatment products used normally. In the group using non-inventive composition, this was only 30%.

The above results clearly show the anti-fat effect of the hair and scalp treatment composition according to the present invention.

Similar results are observed with the following compositions.

### Example 2

| **Vitalizing hair and scalp lotion** | |
|---|---|
| Polyquaternium - 37* | 0.3 % by weight |
| Ethanol | 20 |
| 5-α-avocuta | 0.3 |
| Methyl lactate | 0.2 |
| Cremophor RH 40 | 0.3 |
| Glycerin | 0.5 |
| Water | q.s. to 100 |

| | |
|---|---|
| *: pure in powder from, trade name Ultragel - 300. | |

Polyquaternium-37 is dissolved in sufficient amount of water and combined with 5-α-avocuta, mentyl lactate and Cremophor RH 40 (solubilizer) mixture and finally ethanol, glycerin is added and is made up to 100% with water.

### Example 3

| **Hair and scalp lotion** | |
|---|---|
| Hydroxyethylcellulose | 0.5 % by weight |
| Ethanol | 10 |
| 5-α-avocuta | 0.3 |
| Fragrance | 0.1 |
| Cremophor RH 40 | 0.3 |
| Propyleneglycol | 0.5 |
| Preservative | q.s |
| Citric acid/sodium hydroxide | q.s. to pH 5.5 |
| Water | q.s. to 100 |

Hydroxyethylcellulose is dissolved in sufficient amount of water and combined with 5-α-avocuta, fragrance and Cremophor RH 40 (solubilizer) mixture and finally ethanol, propyleneglycol and preservative is added and after adjustment of pH composition is made up to 100% with water.

### Example 4

| **Hair and scalp lotion** | |
|---|---|
| Carbomer | 0.5 % by weight |
| Ethanol | 25 |
| 5-α-avocuta | 0.5 |
| Cremophor RH 40 | 0.3 |
| Panthenol | 0.5 |
| Sodium hydroxide | q.s. to pH 6.0 |
| Water | q.s. to 100 |

Carbomer is dispersed in sufficient amount of water and neutralized with sufficient amount of sodium hydroxide (pH 6.0) and combined with 5-α-avocuta, and Cremophor RH 40 (solubilizer) mixture and finally ethanol and panthenol are added and is made up to 100% with water.

### Example 5

| **Conditioning Hair and scalp lotion** | |
|---|---|
| Guar hydroxypropyltrimonium chloride | 0.3 % by weight |
| Cetrimonium chloride | 0.2 |
| 5-α-avocuta | 0.4 |
| Cremophor RH 40 | 0.4 |
| Panthenol | 0.5 |
| Citric acid | q.s. to pH 6.0 |
| Water | q.s. to 100 |

### Example 6

| **Hair and scalp lotion with additional antidandruff effect** | |
|---|---|
| Guar hydroxypropyltrimonium chloride | 0.3 % by weight |
| Cetrimonium chloride | 0.2 |
| 5-α-avocuta | 0.4 |
| Cremophor RH 40 | 0.4 |
| Octopirox | 0.1 |
| Panthenol | 0.5 |
| Citric acid | q.s. to pH 6.0 |
| Water | q.s. to 100 |

### Example 7

| **Hair and scalp lotion with additional UV filter** | |
|---|---|
| Guar hydroxypropyltrimonium chloride | 0.3 % by weight |
| Cetrimonium chloride | 0.2 |
| 5-α-avocuta | 0.4 |
| Cremophor RH 40 | 0.4 |
| Benzophenone - 4 | 0.2 |
| Panthenol | 0.5 |
| Citric acid | q.s. to pH 6.0 |
| Water | q.s. to 100 |

### Example 8

| **Aerosol hair and scalp lotion** | |
|---|---|
| Acrylates copolymer | 0.5 % by weight |
| Ethanol | 10 |
| 5-α-avocuta | 0.4 |
| Cremophor RH 40 | 0.4 |
| Panthenol | 0.5 |
| Sodium hydroxide | q.s. to pH 6.0 |
| Water | q.s. to 100 |

The composition is filled in an aerosol can with 6% by weight dimethylether - n-butane (80:20 by weight) propellant mixture and applied onto scalp through an activator having an extended nozzle of around 5 cm.

### Example 9

| **Anti-fat Kit** | |
|---|---|
| **Component A (Cleansing composition)** | |
| Sodium laureth sulfate | 8.0% by weight |
| Decyl polyglucoside | 4.0% |
| Cocamidopropyl betaine | 2.0% |
| Polyquaternium - 7 | 0.5% |
| 5-α-avocuta | 0.5 |
| Cremophor RH 40 | 0.5 |
| Panthenol | 0.5 |
| Citric acid/sodium hydroxide | q.s. pH 5.5 |
| Sodium chloride | q.s. Viscosity 2000 mPa.s |
| Preservative | q.s. |
| Water | q.s. 100 |

| **Component B (Hair and scalp lotion)** | |
|---|---|
| Guar hydroxypropyltrimonium chloride | 0.3 % by weight |
| Oatmeal extract | 1.0 |
| 5-α-avocuta | 0.4 |
| Cremophor RH 40 | 0.4 |
| Panthenol | 0.5 |
| Citric acid | q.s. to pH 6.0 |
| Water | q.s. to 100 |

The compositions are tested for demonstrating their anti fat effect in a use test with 10 end users admitting having greasy hair and using normally anti-fat hair and scalp cleansing and treatment products claiming the anti-fat effect. In a control group quantitatively the same compositions as above but not containing 5-α-Avocuta is tested in the same way. All participants, male, aging 20 to 45, 10 in each group, are asked to use the hair and scalp cleansing and treatment compositions for a period of 3 weeks. Prior to the test period their washing habits are evaluated. Average hair and scalp washing frequency is 6 in a week. The test persons are asked to continue using their own shampoo (hair cleansing products).

After 3 week of usage, average wash frequency in the group using the composition according to the example 6 was dropped to 3 in a week on average (most of the test persons answered the question as every other day) whereas in the group using the composition not containing 5-α-Avocuta (non-inventive composition) did not change at all, the average number was 6 (most of the test persons answered the question as every day). In the group using the inventive composition, preference was approximately 80% towards the inventive composition when compared to hair and scalp treatment products used normally. In the group using non-inventive composition, this was only 20%.

The above results clearly show the anti-fat effect of the hair and scalp treatment composition according to the present invention.

## Claims

1. Hair and scalp treatment composition **characterized in that** it comprises butyl esters of fatty acids of avocado oil one or more thickening polymer, and one or more moisturising agent selected from polyols.

2. Hair and scalp treatment composition according to claim 1 **characterized in that** the concentration of butyl esters of fatty acids of avocado oil is from 0.01 to 5% by weigh calculated to total composition.

3. Hair and scalp treatment composition according to claims 1 and 2 **characterized in that** one or more thickening polymer(s) is/are selected from anionic, non-ionic, cationic and/or amphoteric ones or their mixtures.

4. Hair and scalp treatment composition according to any of the preceding claims **characterized in that** it comprises one or more thickening polymer(s) at a concentration of 0.01 - 5% by weight calculated to total composition

5. Hair and scalp treatment composition according to any of the preceding claims **characterized in that** the thickening polymer is an anionic acrylate polymer.

6. Hair and scalp treatment composition according to any of the preceding claims **characterized in that** it comprises additionally one or more antidandruff agent.

7. Hair and scalp treatment composition according to any of the preceding claims **characterized in that** it comprises additionally one or more extract derived from natural sources.

8. Hair and scalp treatment composition according to claim 7 **characterized in that** extract derived from natural source is oatmeal extract.

9. Hair and scalp treatment composition according to any of the preceding claims **characterized in that** it comprises additionally one or more hair conditioning agent.

10. Hair and scalp treatment composition according to any of the preceding claims **characterized in that** it comprises additionally one or more organic solvent.

11. Hair and scalp treatment composition according to any of the preceding claims **characterized in that** it comprises additionally menthol or menthyl lactate.

12. Hair and scalp treatment composition according to any of the preceding claims **characterized in that** it comprises additionally one or more UV filter.

13. Hair and scalp treatment composition according to any of the preceding claims **characterized in that** it has a viscosity of below 2500 mPa.s measured at 20°C with Brookfield or Höppler viscosimeters at a share rate of 10 sec⁻¹.

14. Hair and scalp treatment kit **characterized in that** comprising at least two components component A and B and component A is being the cleansing composition comprising at least one surfactant selected form anionic, nonionic and amphoteric ones and at least one hair conditioning ingredient and optionally comprises 5-a-avocuta and component B is being a hair and scalp treatment composition according to claims 1 - 13.

15. Process for applying kit according to claim 14 **characterized in that** first component A is applied on dry or wet hair and left on hair and scalp for a period of 1 to 20 min, and rinsed off with water and subsequently component B being a hair and scalp treatment composition according to claims 1 to 13 is applied onto scalp and hair and not rinsed off.

16. Use of compositions according to claims 1 to 13 for reducing greasiness of hair and scalp.

17. Use of kit according to claim 14 for reducing greasiness of hair and scalp.

## Patentansprüche

1. Haar- und Kopfhautbehandlungsmittel, **dadurch gekennzeichnet, dass** es Butylester von Fettsäuren von Avocadoöl, ein oder mehrere verdickende Polymere und ein oder mehrere Feuchtigkeit spendende Mittel, ausgewählt aus Polyolen, enthält.

2. Haar- und Kopfhautbehandlungsmittel nach Anspruch 1, **dadurch gekennzeichnet, dass** die Menge des Butylesters von Fettsäuren des Avocadoöls 0,01 bis 5 Gew.- % beträgt, berechnet auf die gesamte Zusammensetzung.

3. Haar- und Kopfhautbehandlungsmittel nach den Ansprüchen 1 und 2, **dadurch gekennzeichnet, dass** ein oder mehrere Verdickungspolymer(e) aus anionischen, nicht ionischen, kationischen und/oder amphoteren oder deren Mischungen ausgewählt werden.

4. Haar- und Kopfhautbehandlungsmittel nach einem der vorhergehenden Ansprüche, **dadurch gekennzeichnet, dass** es ein oder mehrere Verdickungspolymer(e) in einer Menge von 0,01 - 5 Gew- %, berechnet auf die Gesamtzusammensetzung, enthält.

5. Haar- und Kopfhautbehandlungsmittel nach einem der vorhergehenden Ansprüche, **dadurch gekennzeichnet, dass** das Verdickungspolymer ein anionisches Akrylatpolymer ist.

6. Haar- und Kopfhautbehandlungsmittel nach einem der vorhergehenden Ansprüche, **dadurch gekennzeichnet, dass** es zusätzlich ein oder mehrere Antischuppenmittel enthält.

7. Haar- und Kopfhautbehandlungsmittel nach einem der vorhergehenden Ansprüche, **dadurch gekennzeichnet, dass** es zusätzlich einen oder mehrere Extrakte, gewonnen aus natürlichen Ressourcen, enthält.

8. Haar- und Kopfhautbehandlungsmittel nach Anspruch 7, **dadurch gekennzeichnet, dass** der aus natürlichen Ressourcen hergestellte Extrakt Haferflockenextrakt ist.

9. Haar- und Kopfhautbehandlungsmittel nach einem der vorhergehenden Ansprüche, **dadurch gekennzeichnet, dass** es zusätzlich ein oder mehrere Haarkonditionierungsmittel enthält.

10. Haar- und Kopfhautbehandlungsmittel nach einem der vorhergehenden Ansprüche, **dadurch gekennzeichnet, dass** es zusätzlich ein oder mehrere organische Lösungsmittel enthält.

11. Haar- und Kopfhautbehandlungsmittel nach einem der vorhergehenden Ansprüche, **dadurch gekennzeichnet, dass** es zusätzlich Menthol oder Menthyllactat enthält.

12. Haar- und Kopfhautbehandlungsmittel nach einem der vorhergehenden Ansprüche, **dadurch gekennzeichnet, dass** es zusätzlich einen oder mehrere UV- Filter enthält.

13. Haar- und Kopfhautbehandlungsmittel nach einem der vorhergehenden Ansprüche, **dadurch gekennzeichnet, dass** es eine Viskosität unter 2500 mPa.s hat, gemessen mit einem Brookfield oder Höppler Viskosimeter bei 20°C mit einer Share- Rate von 10 sec⁻¹.

14. Haar- und Kopfhautbehandlungs- Kit, **dadurch gekennzeichnet, dass** es mindestens 2 Komponenten, A und B enthält, wobei Komponente A das Reinigungsmittel ist das mindestens ein Tensid ausgewählt aus einem anionischen, nicht ionischen und amphoteren Tensid und mindestens ein Haarkonditionierungsmittel und optional 5-a-Avocuta enthält und Komponente B ein Haar- und Kopfhautbehandlungsmittel nach den Ansprüchen 1 - 13 ist.

15. Verfahren zum Auftragen eines Kit nach Anspruch 14, **dadurch gekennzeichnet, dass** die Komponente A zuerst auf trockenes oder feuchtes Haar aufgetragen wird und auf dem Haar und der Kopfhaut über einen Zeitraum von 1 bis 20 Minuten bleibt, und mit Wasser ausgespült wird und anschließend die Komponente B, welche ein Haar- und Kopfhautbehandlungsmittel nach den Ansprüchen 1 bis 13 ist auf Kopfhaut und Haar aufgetragen wird und nicht abgespült wird.

16. Verwendung von Zusammensetzungen nach den Ansprüchen 1 bis 13 zum Reduzieren von Nachfetten und Schmieren von Haar und Kopfhaut.

17. Verwendung eines Kit nach Anspruch 14 zur Reduktion von Nachfetten und Schmieren von Haar und Kopfhaut.

## Revendications

1. Composition de traitement des cheveux et du cuir chevelu **caractérisée en ce qu'**elle comprend des esters butyliques d'acides gras d'huile d'avocat, un ou plusieurs polymère(s) épaississant(s), et un ou plusieurs agent(s) hydratant(s) choisi(s) parmi des polyols.

2. Composition de traitement des cheveux et du cuir chevelu selon la revendication 1 **caractérisée en ce que** la concentration d'esters butyliques d'acides gras d'huile d'avocat est de 0,01 à 5% en poids calculée sur la composition totale.

3. Composition de traitement des cheveux et du cuir chevelu selon les revendications 1 et 2 **caractérisée en ce qu'**un ou plusieurs polymère(s) épaississant(s) est/sont choisi(s) parmi des polymères anioniques, non ioniques, cationiques et/ou amphotères ou leurs mélanges.

4. Composition de traitement des cheveux et du cuir chevelu selon l'une quelconque des revendications précédentes **caractérisée en ce qu'**elle comprend un ou plusieurs polymère(s) épaississant(s) à une concentration de 0,01 à 5% en poids calculée sur la composition totale.

5. Composition de traitement des cheveux et du cuir chevelu selon l'une quelconque des revendications précédentes **caractérisée en ce que** le polymère épaississant est un polymère d'acrylate anionique.

6. Composition de traitement des cheveux et du cuir chevelu selon l'une quelconque des revendications précédentes **caractérisée en ce qu'**elle comprend en outre un ou plusieurs agent(s) antipelliculaire(s).

7. Composition de traitement des cheveux et du cuir chevelu selon l'une quelconque des revendications précédentes **caractérisée en ce qu'**elle comprend en outre un ou plusieurs extrait(s) provenant de sources naturelles.

8. Composition de traitement des cheveux et du cuir chevelu selon la revendication 7 **caractérisée en ce que** l'extrait provenant d'une source naturelle est un extrait de farine d'avoine.

9. Composition de traitement des cheveux et du cuir chevelu selon l'une quelconque des revendications précédentes **caractérisée en ce qu'**elle comprend en outre un ou plusieurs agent(s) de conditionnement des cheveux.

10. Composition de traitement des cheveux et du cuir chevelu selon l'une quelconque des revendications précédentes **caractérisée en ce qu'**elle comprend en outre un ou plusieurs solvant(s) organique(s).

11. Composition de traitement des cheveux et du cuir chevelu selon l'une quelconque des revendications précédentes **caractérisée en ce qu'**elle comprend en outre du menthol ou du lactate de menthyle.

12. Composition de traitement des cheveux et du cuir chevelu selon l'une quelconque des revendications précédentes **caractérisée en ce qu'**elle comprend en outre un ou plusieurs filtre(s) anti-UV.

13. Composition de traitement des cheveux et du cuir chevelu selon l'une quelconque des revendications précédentes **caractérisée en ce qu'**elle a une viscosité inférieure à 2 500 mPa.s mesurée à 20°C avec des viscosimètres de Brookfield ou de Höppler à un taux de cisaillement de 10 s⁻¹.

14. Kit de traitement des cheveux et du cuir chevelu **caractérisé en ce que** comprenant au moins deux composants A et B, et le composant A est la composition de nettoyage comprenant au moins un agent tensioactif choisi parmi des agents tensioactifs anioniques, non ioniques et amphotères et au moins un ingrédient de conditionnement des cheveux et comprend facultativement du 5-α-Avocuta et le composant B est une composition de traitement des cheveux et du cuir chevelu selon les revendications 1 à 13.

15. Procédé d'application d'un kit selon la revendication 14 **caractérisé en ce que** le premier composant A est appliqué sur les cheveux secs ou humides et laissé sur les cheveux et le cuir chevelu sur une période de 1 à 20 minutes, et rincé à l'eau et ensuite le composant B étant une composition de traitement des cheveux et du cuir chevelu selon les revendications 1 à 13 est appliqué sur le cuir chevelu et les cheveux et n'est pas rincé.

16. Utilisation des compositions selon les revendications 1 à 13 pour réduire le caractère gras des cheveux et du cuir chevelu.

17. Utilisation d'un kit selon la revendication 14 pour réduire le caractère gras des cheveux et du cuir chevelu.
